# EUROPEAN PATENT APPLICATION

(11) **EP 3 888 629 A1**
(43) Date of publication of application: **06.10.2021**
(21) Application number: 20167280.5
(22) Date of filing: 31.03.2020
(51) Int. Cl.: A61K 9/00, A61K 33/30, A61K 9/70, A61K 47/36, A61K 47/38, A61K 47/32, A61P 31/14

(54) **COMPOSITION FOR TREATMENT OR PREVENTION OF A VIRUS INFECTION**

(71) Applicant: Dr. Kurt Wolff GmbH & Co. KG, 33611 Bielefeld (DE)
(72) Inventor: SCHULZE zur WIESCHE, Erik, 33611 Bielefeld (DE); MEYER, Frederik, 33611 Bielefeld (DE); ENAX, Joachim, 33611 Bielefeld (DE)
(74) Representative: Ter Meer Steinmeister & Partner

(57) **Abstract**

The invention relates to the treatment or prevention of a virus infection.

## Description

### Field of the invention

The present invention is directed to a composition comprising 0.001 to 20 wt.-% of a water-soluble zinc salt and a film-forming polymer for use in the treatment or prevention of a virus infection.

### Background of the invention

Viruses belonging to the family of *Coronaviridae* were first characterized in the 1960's. Virus infections belonging to the family are common in all mammal species including humans. The SARS-CoV-2 outbreak made it evident that novel pharmaceutical compositions are needed to treat and prevent the widespread of virus infections in general, and in particular infections with the SARS-CoV-2.

Corona viruses are RNA viruses with a linear ss-RNA (single-stranded RNA) that are transmitted via droplet infection and result in fever and respiratory problems. There are several species that are relatively harmless and result in only acute and mild symptoms or take an asymptomatic course (HCoV-OC43, 229E, HKU1, and HL63). On the other hand, SARS-CoV (Severe Acute Respiratory Syndrome Corona Virus), MERS-CoV (Middle East Respiratory Syndrome Corona Virus) and SARS-CoV2 are Corona viruses that can lead to severe respiratory and lung infections. So far there is neither a treatment nor prophylaxis, except for exposure prophylaxis.

Therefore, it was an object of the present invention to provide a novel, safe, and effective composition for the treatment or prevention of a virus infection in general and in particular for the treatment or prevention of SARS-CoV-2.

### Summary of the invention

These objects have surprisingly been solved by a composition comprising 0.001 to 20 wt.-% of a water-soluble zinc salt and a film-forming polymer for use in the treatment or prevention of a virus infection,
wherein the water-soluble zinc salt is selected from the group consisting of zinc-L-pyrrolidone-carboxylate, zinc acetate, zinc chloride, zinc histidine, zinc gluconate, zinc aspartate, zinc citrate, zinc sulfate, zinc lactate and mixtures thereof, and
wherein the film-forming polymer is selected from the group consisting of carrageenan, carboxymethyl cellulose, hyaluronic acid and pharmaceutically acceptable salts thereof, copolymers of methyl vinyl ether and maleic acid, and pharmaceutically acceptable salts thereof, carboxymethyl chitin, polyvinylpyrrolidone, chitosan, hydroxypropyl cellulose, and mixtures thereof.

It has surprisingly been found that according to the above aspect, infections with a virus can be prevented or the probability of infection can at least significantly be reduced. In addition, and in accordance with the above aspect, the virus count in the respiratory tract is significantly reduced so that an infected person is no longer infectious towards third persons.

Without being bound to theory, it is believed that the present invention functions by forming a protective layer of the composition according to the present invention on the mucous membrane in the mouth and/or nose. This antiviral drug protective layer effectively prevents infection with a virus by droplet infection in subjects not yet infected. In addition, the composition also reduces the virus count in the respiratory tract and in addition, a protective layer is formed so that a subject already infected is less infectious to other subjects, thereby also preventing further infections.

In another aspect, the present invention relates to a composition comprising
0.001 to 20 wt.-% of a water-soluble zinc salt selected from the group consisting of zinc-L-pyrrolidone-carboxylate, zinc acetate, zinc chloride, zinc histidine, zinc gluconate, zinc aspartate, zinc citrate, zinc sulfate, zinc lactate and mixtures thereof, and mixtures thereof,
0.001 to 20 wt.-% of a water-insoluble zinc salt selected from the group consisting of zinc hydroxyapatite, zinc oxide, and mixtures thereof, and
a film-forming polymer selected from the group consisting carrageenan, carboxymethyl cellulose, hyaluronic acid and pharmaceutically acceptable salts thereof, copolymers of methyl vinyl ether and maleic acid, and pharmaceutically acceptable salts thereof, carboxymethyl chitin, polyvinylpyrrolidone, chitosan, hydroxypropyl cellulose, and mixtures thereof.

The present invention also relates to a kit of parts comprising the composition of the present invention and a spraying device for applying the composition to the mucous membrane.

### Detailed description

The following definitions are relevant in connection with the embodiments of the present invention.

The meaning of the term "comprising" is to be interpreted as encompassing all the specifically mentioned features as well optional, additional, unspecified ones, whereas the term "consisting of" only includes those features as specified. Therefore, "comprising" includes as a limiting case the composition specified by "consisting of".

The term "weight-%" or "wt.-%" is to be understood to refer to the weight amount of the component relative to the total weight amount of the respective composition, if not specified otherwise.

The term "water-soluble" refers to a zinc salt that has a solubility of 0.1 g/L or more at standard conditions. Correspondingly, the term "water-insoluble" refers to a zinc-salt that has a solubility of less than 0.1 g/L at standard conditions, i.e. at a temperature of 15 °C and a pH value of 7.0.

The term "film-forming" polymer refers to a polymer that results in a cohesive, continuous layer when applied to a surface. In the present invention, when the composition is applied topically to the mucous membrane, the film-forming polymer leads to the formation of a cohesive layer of the composition that further has enhanced adhesive properties to the mucous membrane.

As used herein, the term "prevention" of a virus infection does not only refer to prevention of an infection for the subject that uses the composition of the present invention but also refers to the prevention of passing the infection on to further subjects, in case the subject that uses the composition is already infected with the virus. Thus, a dual method of preventing widespread of the virus is achieved by the composition of the present invention. Additionally, the composition has viral activity so that the virus count is decreased in the throat and nasal area, thereby resulting in an effective local treatment of the virus, as the virus is predominately located in these areas.

Preferred embodiments according to the invention are defined hereinafter. The preferred embodiments are preferred alone or in combination. Further, it is to be understood that the following preferred embodiments refer to all aspects of the present invention, i.e. the compound for use, the compound, and the kit of parts.

In an embodiment, the invention relates to a composition comprising 0.001 to 20 wt.-% of a water-soluble zinc salt and a film-forming polymer for use in the treatment or prevention of a virus infection,
wherein the water-soluble zinc salt is selected from the group consisting of zinc-L-pyrrolidone-carboxylate, zinc acetate, zinc chloride, zinc histidine, zinc gluconate, zinc aspartate, zinc citrate, zinc sulfate, zinc lactate and mixtures thereof, and
wherein the film-forming polymer is selected from the group consisting of carrageenan, carboxymethyl cellulose, hyaluronic acid and pharmaceutically acceptable salts thereof, copolymers of methyl vinyl ether and maleic acid, and pharmaceutically acceptable salts thereof, carboxymethyl chitin, polyvinylpyrrolidone, chitosan, hydroxypropyl cellulose, and mixtures thereof.

The above composition is useful for both, the treatment as well as the prevention of a virus infection. In a preferred embodiment, the composition is used for the prevention of a virus infection.

The water-soluble zinc salt also encompasses solvates (such as hydrates) of the respective zinc salts. It is preferred that the composition comprises water and is an aqueous solution, suspension or emulsion. Therefore, it is not particularly relevant which specific solvate is used.

In a preferred embodiment, the composition comprises the water-soluble zinc salt in an amount of 0.01 to 10 wt.-%, 0.05 to 5 wt.-% or 0.05 to 1 wt.-%, preferably in an amount of 0.05 to 0.5 wt.-%.

The copolymer of polyvinyl ether and maleic acid is preferable an alternating copolymer. It is to be understood that the polymer is derived from monomer units of methyl vinyl ether and maleic acid or maleic anhydride and the copolymer is also called poly(methyl vinyl ether-co-maleic acid). This polymer can be obtained by e.g. radical polymerization of the monomer units and copolymers of methyl vinyl ether and maleic acid are commercially available as Gantrez™ S polymer (International Nomenclature of Cosmetic Ingredients: PVM/MA copolymer; Ashland Inc.).

Pharmaceutically acceptable salts of this copolymer can be alkali metals and/or earth alkali metals. Thus, the salts can be selected from lithium, sodium, potassium, magnesium, calcium, and mixtures thereof. It is preferred that the salt is a sodium/calcium salt. Calcium sodium copolymer of poly(methyl vinyl ether-co-maleic acid) are commercially available as Gantrez™ MS-955 polymer (International Nomenclature of Cosmetic Ingredients: calcium/sodium PVM/MA copolymer Ashland Inc.).

The carboxymethyl cellulose, also referred to as cellulose gum, is a polycarboxymethyl ether of cellulose. It is to be understood that the term "carboxymethyl cellulose" also encompasses derivatives and/or pharmaceutically acceptable salts, such as salts selected from calcium, sodium, potassium, and mixtures thereof.

In a preferred embodiment, the composition contains a combination of at least two zinc salts.

The composition may additionally contain a flavoring agent. Non-limiting examples of flavoring agents are sweeteners and aromatic oils, Sweeteners can be sugar alcohols and/or disaccharides (mannitol, sorbitol, xylitol, isomalt, glucose, fructose, maltose), aspartame, saccharin, and corn syrup. Non-limiting examples of aromatic oils include menthol, peppermint oil, and spearmint oil.

Pharmaceutically acceptable salts of hyaluronic acid comprise the earth metal salts, such as the sodium and potassium salt.

In an embodiment, the composition comprises the film forming polymer in an amount of 0.01 to 10 wt.-%, 0.1 to 5 wt.-% or 1 to 2 wt.-%, preferably in an amount of 0.5 to 3 wt.-%.

In an embodiment, the composition further comprising a water-insoluble zinc salt. In an embodiment, the water-insoluble salt is present in an amount of 0.001 to 20 wt.-%. In a preferred embodiment, the composition comprises the water-insoluble zinc salt in an amount of 0.01 to 10 wt.-%, 0.05 to 5 wt.-% or 0.05 to 1 wt.-%, preferably in an amount of 0.05 to 0.5 wt.-%.

In yet another embodiment, the water-insoluble zinc salt is selected from the group consisting of zinc hydroxyapatite, zinc oxide, and mixtures thereof.

In an embodiment, the composition further comprises an additional active ingredient selected from dexpanthenol, ectoine, menthol, lactoferrin, xylitol, saccharin, and mixtures thereof. These ingredients can have anti-inflammatory properties and/or protective properties of the skin and further enhance treatment or prevention of a virus infection. These additional active ingredients are preferably present in an amount of 0.001 to 10 wt.-%, 0.01 to 5 or 0.05 to 2 wt.-%.

In another embodiment, the composition may comprise preservatives, such as benzoic acid, citric acid, lactic acid, ascorbic acid, tocopherol, as well as pharmaceutically acceptable salts thereof.

In another embodiment, the composition can further comprise a surfactant. In a preferred embodiment, the composition further comprises a surfactant selected from alkyl sulfates, alkyl sarcosinates, alkyl taurates, and mixtures thereof.

Alkyl sulfates have the formula ROSO₃M, alkyl sarcosinates have the formula RC(O)N(CH₃)CH₂CO₂M, and taurates have the formula RC(O)N(CH₃)CH₂CH₂SO₃M, wherein R is a C₄-C₂₆ alkyl or C₄-C₂₆ alkenyl, and M is a water-soluble cation such as ammonium, sodium, potassium or triethanolamine. Preferably, R is C₁₂-C₁₆ alkyl or C₁₂-C₁₈.

In an embodiment, the composition is administered as an orally disintegrating tablet, a lozenge, as mouth rinse or as an aerosol by spraying. In an embodiment, the composition is applied topically by spraying the composition on the respective area. In order to form a uniform, homogenous and long-lasting protective layer on the mucous membrane, it is preferred that the composition is administered by spraying the composition. In addition, by spraying the composition, a protective layer can be formed on a larger area, thereby leading to an even better protection.

In an embodiment, the composition is applied to the mucous membrane, preferable using a spraying device.

The spraying device can be used to apply the composition to the skin of e.g. the hands or to the mucous membrane in the mouth, pharynx or nose. It is preferred that the spraying device is used to apply the composition to the mucous membrane in the mouth or pharynx. It is particularly preferred that the spraying device contains a means for (pre-)determining the amount of the composition that is applied.

Suitable pump spraying devices are known (see i.a. US 4,245,967) and are commercially available as "throat sprays". Such sprays are usually used to treat sore throats caused by bacterial infections of the throat and mucous membrane and/or decrease any symptoms of such infections. Such sprays can contain an antibacterial ingredient to fight the bacterial infection as well as an analgesic to decrease any symptoms of pain and/or soreness.

Spraying devices commonly comprise a feed chamber and an outlet through which the composition is sprayed in a predetermined amount by a suitable means for spraying the composition through a nozzle. It is preferred that the spraying device is a pump spraying device. Such a pump spray contains an atomizer nozzle that disperses the liquid composition as a fine aerosol. This aerosol covers the area on which it is sprayed and forms a thin film.

In an embodiment, the virus belongs to the family of *Coronaviridae,* and preferably is SARS-CoV-2. In another embodiment, the virus belongs to the family of *Orthomyxoviridae, Paramyxoviridae, Filoviridae,* or *Picornaviridae.*

### Examples

### Example 1:

A composition comprising the components as depicted below in Table 1 was obtained by mixing the components.

Epithelial cultures of oral mucosa, fibroblasts, and nasopharyngeal explants which were all generated from patient-derived tissue specimen harvested during medically-indicated surgeries, were provided.

In order to simulate a coronaviral infection in epithelial and fibroblastic cell populations in vitro, poly(I:C) and R 848 (Resiquimod) were be administered at different doses between 1 ng/ml and 10µg/ml to determine the optimal dose to measure inflammatory and immune-modulatory cell signaling pathways in primary cell and tissue cultures. At the same time, the compositions of Examples 1 and 2 were assayed for their biocompatibility. It was found that the compositions are effective.

**Table 1: Ingredients of Example 1**

| **Ingredients** | **Amount in wt.- %** |
|---|---|
| Aqua | add.100 |
| Carrageenan | 0.2 |
| Zinc PCA | 0.1 |
| Zinc Acetate | 0.1 |
| Dexpanthenol | 0.1 |
| Sodium Hyaluronate | 0.05 |
| Sodium Benzoate | 0.3 |
| Citric Acid | q.s. |

### Example 2:

A composition comprising the components as depicted below in Table 1 was obtained by mixing the components.

**Table 2: Ingredients of Example 2**

| **Ingredients** | **Amount in wt.-%** |
|---|---|
| Aqua | add.100 |
| Sorbitol | 0.2 |
| Zinc PCA | 0.1 |
| Xylitol | 0.1 |
| Dexpanthenol | 0.1 |
| Sodium Hyaluronate | 0.05 |
| Sodium Benzoate | 0.3 |
| Zinc Hydroxy-apatite | 2.0 |
| Cellulose Gum | 1.5 |
| Sodium Saccharin | 0.2 |
| Sodium Methyl Cocoyl Taurate | 1.0 |
| Sodium Myristoyl Sarcosinate | 1.0 |

## Claims

1. A composition comprising 0.001 to 20 wt.-% of a water-soluble zinc salt and a film-forming polymer for use in the treatment or prevention of a virus infection,
wherein the water-soluble zinc salt is selected from the group consisting of zinc-L-pyrrolidone-carboxylate, zinc acetate, zinc chloride, zinc histidine, zinc gluconate, zinc aspartate, zinc citrate, zinc sulfate, zinc lactate and mixtures thereof, and
wherein the film-forming polymer is selected from the group consisting of carrageenan, carboxymethyl cellulose, hyaluronic acid and pharmaceutically acceptable salts thereof, copolymers of methyl vinyl ether and maleic acid, and pharmaceutically acceptable salts thereof, carboxymethyl chitin, polyvinylpyrrolidone, chitosan, hydroxypropyl cellulose, and mixtures thereof.

2. The composition for use according to claim 1, further comprising a water-insoluble zinc salt.

3. The composition for use according to claim 2, wherein the water-insoluble salt is present in an amount of 0.001 to 20 wt.-%.

4. The composition for use according to claim 2 or 3, wherein the water-insoluble zinc salt is selected from the group consisting of zinc hydroxyapatite, zinc oxide, and mixtures thereof.

5. The composition for use according to any one of claims 1 to 4, further comprising an additional active ingredient selected from dexpanthenol, ectoine, menthol, lactoferrin, xylitol, saccharin, and mixtures thereof.

6. The composition for use according to any one of claims 1 to 5, further comprising a surfactant selected from alkyl sulfates, alkyl sarcosinates, alkyl taurates, and mixtures thereof.

7. The composition for use according to any one of claims 1 to 6, wherein the composition is administered as an orally disintegrating tablet, as mouth rinse or as an aerosol by spraying.

8. The composition for use according to any one of claims 1 to 7, wherein the composition is applied to the mucous membrane, preferable using a spraying device.

9. The composition for use according to any one of claims 1 to 8, wherein the virus belongs to the family of *Coronaviridae,* and preferably is SARS-CoV-2.

10. A composition comprising 0.001 to 20 wt.-% of a water-soluble zinc salt selected from the group consisting of zinc-L-pyrrolidone-carboxylate, zinc acetate, zinc chloride, zinc histidine, zinc gluconate, zinc aspartate, zinc citrate, zinc sulfate, zinc lactate and mixtures thereof, and mixtures thereof, 0.001 to 20 wt.-% of a water-insoluble zinc salt selected from the group consisting of zinc hydroxyapatite, zinc oxide, and mixtures thereof, and
a film-forming polymer selected from the group consisting of carrageenan, carboxymethyl cellulose, hyaluronic acid and pharmaceutically acceptable salts thereof, copolymers of methyl vinyl ether and maleic acid, and pharmaceutically acceptable salts thereof, carboxymethyl chitin, polyvinylpyrrolidone, chitosan, hydroxypropyl cellulose, and mixtures thereof.

11. The composition of claim 10 inhibiting the replication of viral agents on the mucous membrane.

12. A kit of parts comprising the composition for use according to any one of claims 1 to 9 and a spraying device for applying the composition to the mucous membrane.
